# EUROPEAN PATENT SPECIFICATION

(11) **EP 1 230 908 B1**
(45) Date of publication and mention of the grant of the patent: **20.10.2004**
(21) Application number: 00971448.6
(22) Date of filing: 18.10.2000
(51) Int. Cl.: A61K 7/28

(54) **TEETH WHITENING PRODUCT**
ZAHNBLEICHMITTEL
PRODUIT POUR LE BLANCHIMENT DENTAIRE

(30) Priority: 17.11.1999 ES 9902522
(43) Date of publication of application: 14.08.2002
(73) Proprietor: Universitat de les Illes Balears, 07071 Palma de Mallorca (ES); Laboratorios Kin, S.A., 08018 Barcelona (ES)
(72) Inventor: PONS BIESCAS, Antonio, E-07003 Palma de Mallorca (ES); TUR MARI, Josep Antoni, E-07011 Palma de Mallorca (ES); RIUTORD SBERT, Pere, E-07120 Palma de Mallorca (ES); TAULER RIERA, Pedro, E-07021 Felanitx (ES); GIMENO FRANCO, Isabel, E-07610 Sometines (ES); BALASCH RISUENO, Ignacio, E-08022 Barcelona (ES); SANCHO RIERA, Enriqueta, E-08029 Barcelona (ES)
(74) Representative: Curell Aguilà, Marcelino
(86) International application number: PCT/ES2000/000400
(87) International publication number: WO 2001/035919

(56) References cited:
- WO-A-97/06775
- US-A- 4 150 113
- US-A- 4 178 362
- US-A- 4 269 822
- US-A- 4 537 764
- US-A- 4 564 519

## Description

### TECHNICAL FIELD OF THE INVENTION

This invention falls within the technical field of dentifrices and mouth washes and, in particular, it relates to dentifrices and mouth washes capable of dental bleaching.

Some of the dentifrices and mouth washes used for dental bleaching are based on the action of hydrogen peroxide or a hydrogen peroxide precursor as a non-specific bleaching agent. Occasionally, in addition to the actual bleaching agent, relatively high temperatures and even light are used to accelerate the process. To date, no description exists of the use of enzymes to accelerate the de-staining process.

This invention relates to the use of enzymes as catalysts in the de-staining process and can find application in the field of dental bleaching. The enzymes that can be used are those using hydrogen peroxide as one substrate thereof, for which they are specific, being non-specific with respect to the other substrate thereof.

This invention is applicable to the formulation of tooth pastes, mouth washes and similar preparations that can be used in dental surgeries or on the move or as the basis of a daily dental hygiene routine and which are intended to whiten teeth.

### PRIOR ART

At present, dental bleaching techniques are increasingly in demand as a result of today's prevailing aesthetics culture. However, the first publications explaining a dental bleaching process date from the last century. In 1877 the use of oxalic acid as a bleaching agent was published for the first time (Zaragoza, V.M.T. *Bleaching of vital teeth:technique.* Estomodeo. **1984,** *9*, 7-30). The use of hydrogen peroxide as a dental bleaching agent was described for the first time in 1884 (Freinman, R., Goldstein, R., Garber, D. *Bleaching teeth.* Quintessence Int. **1987,** *1, 10).* In 1895 Garretson used chlorine as a bleaching agent for non vital teeth (Garretson, J.E. *A system of oral surgery.* 6^{th} Ed. Philadelphia: JB Lippincott, **1985**) and Walter Kaine used muriatic acid (CIH 18%). In 1918 Abbot rediscovered the use of hydrogen peroxide as a bleaching agent, using a high concentration (35%) and also a light source (Zaragoza, **1984** - see reference above). Throughout the 20^{th} century concentrated hydrogen peroxide has been used for dental bleaching, even for bleaching tetracycline-stained teeth (Cohen, S., Parkins, F.M. *Bleaching tetracycline-stained vital teeth.* Oral Surg. **1970,** *29*, 465-471). At present, home-based treatment using hydrogen peroxide or preparations based thereon in more dilute concentrations not requiring specialist application, has become popular (Haywood, V.B., Heymann, H.O. *Nightguard vital bleaching.* Quintessence Int. **1989**, *20*, 173-176).

In the absence of transition metals hydrogen peroxide is a relatively stable oxidising agent. Therefore, in the absence of transition metals, hydrogen peroxide reacts with some organic compounds, producing alkene epoxidation and hydroxylation reactions, amine and sulphide oxygenation reactions, and the preparation of peroxiacids and hydroperoxiacids from carboxylic acids and other organic compounds; however, hydrogen peroxide does not react with other organic molecules such as alkanes, for example.

The presence of transition metals greatly increases hydrogen peroxide reactivity. In the presence of transition metals such as iron or copper or transition complexes obtained therefrom, such as for example the heme or hemin group or cytochromes, hydrogen peroxide produces radical hydroxyl, an extremely reactive kind capable of oxygenating any organic compound. The increase in hydrogen peroxide reactivity caused by these catalysts is accompanied furthermore by greater reaction selectivity. For example, the reaction of hydrogen peroxide with olefins in the presence of cytochrome P-450, a monoxygenase containing heminic iron, results in the exclusive formation of an epoxide.

The products of peroxidation with hydrogen peroxide are not stable and in many cases they can continue reacting, leading to ruptures in the chain and the formation of colourless compounds. This is the basis of de-staining using hydrogen peroxide. In fact, hydrogen peroxide can be considered to be an initiator in autoxidation processes following a chain reaction diagram. The autoxidation of aldehydes and polyunsaturated compounds such as fatty acids is a common process that modifies the organoleptic and nutritional features of foodstuffs and which is the basis of the development of frequent, widespread pathologies in today's society as is the case of arteriosclerosis, cataracts, some cancers, etc.

The use of high concentrations of hydrogen peroxide (for example, a concentration of 10% carbamide peroxide) for dental bleaching does not affect the enamel texture, although, generally speaking, it causes a series of side effects, which although they are not serious or sufficient enough to exclude the application thereof, they do cause discomfort to users. Bleaching treatments cause greater temporary sensitivity to temperature changes owing to the action of free radicals which tends to disappear after a few days. The treatments also reduce the adhesion of composites and bleaches parts that are not in direct contact with the bleaching solution. The bleaching agent causes gingival irritation, a hairy tongue, and throat irritation and, if unintentionally swallowed, it also irritates the stomach. A major cause of these side effects is precisely the need to use high concentrations of the bleaching agent. Reducing the bleaching agent concentration while maintaining the bleaching effects, would be a worthwhile innovation in the field of dental bleaching.

Enzymes are catalysts of a protein nature, which reduce the active energy required in order that the substrates give rise to the reaction products. Enzymes have a high catalytic capacity, much higher than any chemical catalyst. On the other hand, enzymes generally present a high level of reaction and substrate selectivity, which enables them to direct the selective transformation of substances towards certain products even in the presence of substance mixtures having a very similar structure. This action and substrate specificity can be considered in the sense that some enzymes allow different substrates having certain similar features to enter into their catalytic centre and be subsequently transformed therein. This is the case of proteases, enzymes that specifically catalyse the hydrolysis of peptide bonds but which can use various proteins as their substrate. Therefore, for example, papain added to a bleaching dentifrice can hydrolyse a high number of different proteins that can be found adhered to the tooth. Other enzymes showing non-exclusive specificity characteristics are peroxidases.

The term "peroxidases" comprises a group of specific enzymes (NAD-peroxidase, peroxidase fatty acid, cytochrome-peroxidase, glutation peroxidase, etc.) as well as a group of non-specific enzymes, known simply as peroxidases (EC 1.11.1.7.). The peroxidases catalyse the dehydrogenation of a high number of aromatic organic compounds such as phenols, hydroquinones and hydroquinoid amines. Peroxidases are hemeproteins which catalyse the transfer of hydrogen or electrons from a donor to an acceptor, usually hydrogen peroxide. The peroxidase donor specificity is very low, whilst, it is high for the acceptor, which can only be hydrogen peroxide, or in some cases acetyl, methyl or ethyl hydropropoxide.

Peroxidases can be obtained from various animal or vegetable sources or from micro-organisms such as, for example, radish, pineapple, potato, legumes, tobacco, yeasts and bacteria. Therefore, peroxidases have various enzymatic isoforms.

By virtue of the features of peroxidase catalysed reactions these can be used as bleaching elements for various types of products. Therefore, peroxidases are introduced into the bleaching methods, or use is made of their presence therein for paint, foodstuffs bleaching, detergents, etc.

WO-A-9706775 discloses a composition for bleaching teeth comprising hydrogen peroxide and peroxidase.

### DETAILED DESCRIPTION OF THE INVENTION

This invention relates to the use of peroxidase as a biocatalyst in dental bleaching processes in which carbamide peroxide is used as the bleaching agent.

This invention also relates to the formulation of dentifrices, mouth washes and dental bleaching treatments that use peroxidase as the enzyme in the resulting de-staining reaction. The idea of using peroxidase in dental bleaching treatments is very interesting because the contact time needed between the bleaching agent and the tooth is reduced and more dilute concentrations of the bleaching agent can be used.

Tooth de-staining occurs when the bleaching solution comes into contact with the substance producing the undesired tooth staining. Consequently, a reaction takes place whereby the staining substrates are converted into colourless products. The de-staining process is accelerated in the presence of peroxidase, with the colourless products being produced in less time.

The peroxidase's lack of specificity enables it to catalyse the reaction of one same bleaching agent or acceptor with a variety of donor staining substances, including tetracyclines, one of the main causes of dental staining which is difficult to remove.

This invention also relates to a dental bleaching method that includes the application of a product comprising a carbamide peroxide and peroxidase. However, in each case, the bleaching agent must come into contact with the enzyme at the same time that both come into contact with the tooth. Therefore, the enzyme and the bleaching agent should be kept separate and mixed immediately before they are used for dental bleaching. It is also possible to first apply the bleaching agent in the mouth and, then add immediately the peroxidase or vice versa.

In a preferred embodiment of this invention, said method comprises the stages of:
(a) keeping said bleaching compound and said enzyme separate until the moment the product is applied;
(b) mixing said bleaching compound and said peroxidase immediately before applying the product; and
(c) applying the mixture of said bleaching compound and said peroxidase to the teeth.

In another preferred embodiment of this invention, said method comprises the steps of:
(a) keeping said bleaching compound and said enzyme separate until the moment the product is applied;
(b) first applying said bleaching compound to the teeth; and
(c) immediately applying said peroxidase.

In another preferred embodiment of this invention, said method comprises the steps of:
(a) keeping said bleaching compound and said enzyme separate until the moment the product is applied;
(b) first applying said peroxidase to the teeth; and
(c) immediately applying said bleaching compound.

The most suitable conditions for performing dental bleaching according to a method of the invention, are described below:
BUFFER: it is advisable to buffer the reaction pH value to a range between 4 and 9, preferably 6.5. The most suitable pH value in each particular case will depend on the isoenzymatic form of the peroxidase that is used and the type of staining on the teeth. Various buffering substances can be used, preferably a phosphate buffer. Said buffering substance must be permitted for buccal use. In principle, any buffering substance permitted for buccal use can be used within the scope of this invention, since no inhibiting effects derived from this type of substrates have been described.
BLEACHING AGENT: the bleaching agent is one of the enzyme substrates, the other substrate being the tooth staining substance. The bleaching agent that is used is carbamide peroxide, a urea additive and hydrogen peroxide, which gradually releases hydrogen peroxide, the real enzyme substrate.

The group of hydrogen peroxide precursors includes a whole series of reactions catalysed by enzymes that produce hydrogen peroxide as the main or secondary product. These reactions include those catalysed by enzymes such as glucose oxidase (EC 1.1.3.4), xanthine-hypoxanthine oxidase (EC 1.1.3.22), ascorbate oxidase (EC 1.10.3.3) and other oxidases. In these cases the substrate, enzyme and buffer concentrations can be adjusted so that the production of hydrogen peroxide is coupled with the use thereof by the peroxidase, thus producing optimum dental bleaching.

The concentration of effective hydrogen peroxide must be adjusted in relation to the amount of enzyme present. We have confirmed that a high concentration of hydrogen peroxide inactivates the enzyme. Consequently, it is necessary to adjust the concentration of the enzyme present in line with the concentration of hydrogen peroxide that is used or vice versa. By way of an example of the above affirmation, the following quantities not limiting the scope of this invention, in a 0.09M concentration of carbamide peroxide, the addition of peroxidase up to a final concentration of 40 units pyrogallol/ml increases the speed with which a tetracycline solution (0.015M) is rendered colourless, approximately 2.5 times. On the other hand, in a 0.17M concentration of carbamide peroxide, the addition of 20 units pyrogallol/ml of peroxidase only increases the speed with which a 0.015M tetracycline solution is rendered colourless, approximately 1.3 times.

PEROXIDASE: the peroxidase that can be used for dental bleaching can be obtained following a purification process or directly after it is obtained from its source. This enzyme is widely available in nature, and can be found both in vegetable and animal sources and in micro-organisms. Any peroxidase isoform can be used within the scope of this invention, said peroxidase originating from any vegetable or animal source or micro-organism, radish peroxidase being preferred.

By way of a non-limiting example of this invention, using radish peroxidase (20 units pyrogallol/ml), pH 6.5 buffered with phosphates and a concentration of 1.5% carbamide peroxide it has been possible to increase the brightness factor of a tooth from 64%, before treatment, to 74% after treatment; however, if peroxidase is not present, the same type of treatment at the same time does not alter the tooth's 64% brightness factor.

The peroxidase concentration used in this invention is that which provides a minimum activity of 10 units of pyrogallol, preferably a minimum of 20 units of pyrogallol (one unit of pyrogallol is the quantity of enzyme required to convert 1 mg of pyrogallol into purpurogallin in 20 seconds at pH 6.0 and a temperature of 20°C).

The use of a particular peroxidase implies the prior characterisation of stability, optimum pH, substrate concentration, enzyme concentration, presence of activators and exclusion of inhibitors or inactivators.

INTRODUCTION CARRIER: the introduction carrier used for the bleaching agent, the buffered medium and the enzyme depends on the type of product or technique desired to be used (dentifrice, mouth wash, dental bleachers used in dentist surgeries).

The carrier to be used must not contain enzyme inhibitors or inactivators, on the other hand, it must contain all those substances which can activate the enzyme. The enzyme can be dissolved in the carrier or it can be immobilised or microencapsulated to preserve its stability. Enzyme immobilisation can be carried out by means of the technique of trapping it in gels or polymers, or by crossing it with other proteins or other soluble or insoluble materials in the carrier, or by microencapsulating it.

The way in which the enzyme is introduced into the mouth must correspond to the effect that the particular carrier can have on the enzyme's stability. In this latter case, the inactivating or inhibiting effect that the carrier may cause or the time the enzyme remains in the carrier must be compensated with an initial increase in the amount of enzyme to be used. Therefore, the way in which the peroxidase and the bleaching agents are fed-in must be such that at least a peroxidase activity of a minimum of 10 units of pyrogallol is ensured, preferably a minimum of 20 units of pyrogallol.

### BRIEF DESCRIPTION OF THE DRAWINGS

Figures 1 and 2 are representations of the speed with which tetracycline is rendered colourless depending on the concentration of carbamide peroxide that is present. Figure 1 corresponds to low concentrations of carbamide peroxide, while Figure 2 corresponds to high concentrations of carbamide peroxide.
Figures 3 and 4 are representations of the speed with which tetracyclines is rendered colourless depending on the peroxidase concentration, in the presence of carbamide peroxide at high or low concentrations, respectively.
Figure 5 is a comparative representation of the percentage of time-dependant tooth brightness, corresponding to teeth bleached in the absence of, or in the presence, of enzyme.

### EMBODIMENTS OF THE INVENTION

This invention is best illustrated by means of the following examples. The particular concentrations of enzyme, buffer and bleaching agent described in the examples can be varied to other concentrations. Where a particular peroxidase isoform has been used, other isoforms from any vegetable or animal source or micro-organism can be used. Where a mouth wash has been used, this can be extended to any dentifrice or dental bleaching product that uses peroxidase. Where a carrier having a particular composition has been used, another peroxidase compatible carrier can be used. Where a certain odontology technique for dental bleaching using peroxidase has been used, another different technique, also based on the use of peroxidase, can be used.

### EXAMPLE 1

This example illustrates the action of a mouth wash designed on the basis of using peroxidase to render a tetracycline solution colourless. Also it shows the limitations affecting the concentration of carbamide peroxide effective in rendering tetracycline colourless.

The mouth wash consists of a 2.4% carbamide peroxide solution in 0.1 M phosphate buffer, pH 6.5 and on the other hand, a peroxidase solution (120 units of pyrogallol/ml) in 0.1 M phosphate buffer, pH 6.5 which is fed into the container in which the de-staining reaction is to take place, immediately after the mouth wash.

The substance to be rendered colourless is tetracycline, which is solublised in water at a concentration of 0.015M. The maximum tetracycline absorption wavelength has been selected so as to monitor the de-staining speed. The de-staining speed is determined using a spectrophotometer which allows the absorption to be monitored continually, the reaction tray being thermostated at 37°C. The de-staining reaction speeds have been calculated from the relations between absorbency variation and the reaction time, and they have been expressed in nKat.

The necessary quantities of tetracycline solution, the mouth wash and enzyme (in this order) are placed in the reaction tray to obtain the parameter combinations according to which it is intended to evaluate the enzyme bleaching capacity.

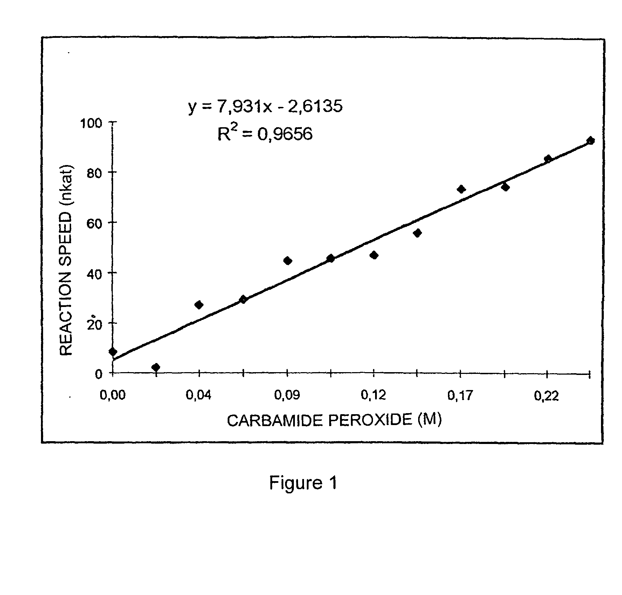

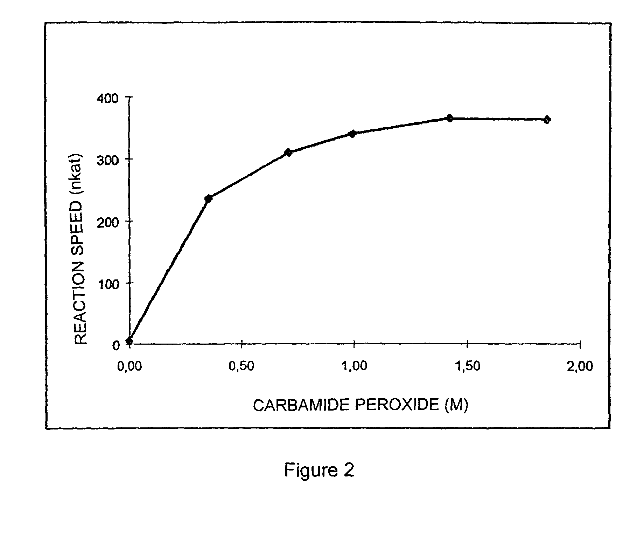

The mouth wash alone renders a tetracycline solution colourless in the absence of peroxidase as represented in Figure 1. The speed with which the tetracycline solution is rendered colourless is directly proportional to the concentration of carbamide peroxide present. This relation follows a first order equation in which the de-staining speed is related to the carbamide peroxide concentration by a straight line inclined at 7.93 nkatxL/mol and an ordinate at origin of -2.6 nkat (lineal regression coefficient =0.9656). However, with high concentrations of carbamide peroxide this lineal relation fails (Figure 2). The relation between the speed with which tetracycline is rendered colourless and the carbamide peroxide concentration is the typical saturation relation from a carbamide peroxide concentration of approximately 0.70M. Once this concentration is obtained, the speed with which tetracycline is rendered colourless does not increase, regardless of how much the carbamide peroxide concentration is increased.

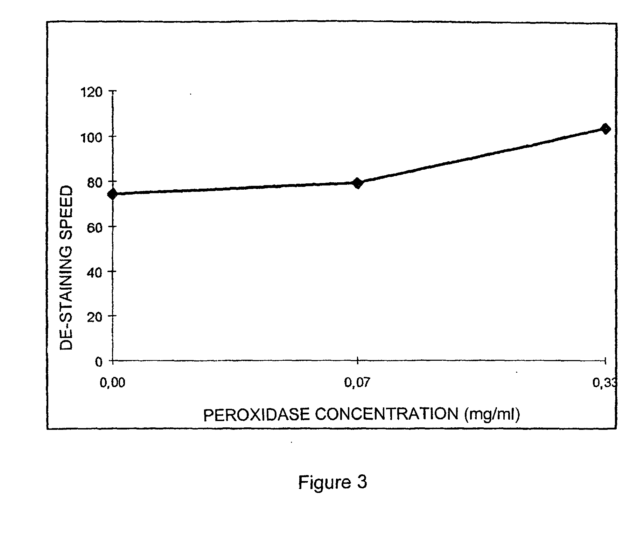

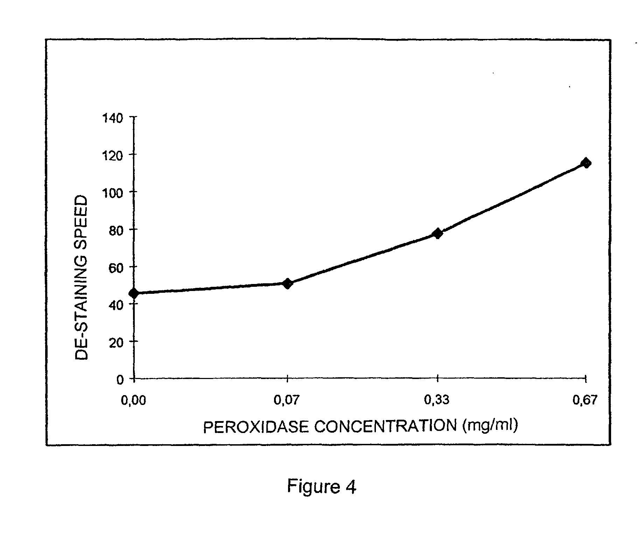

The addition of peroxidase to the mouth wash as it comes into contact with the staining substance increases the de-staining speed which depends on the concentration of enzyme and carbamide peroxide present (Figures 3 and 4). When the carbamide peroxide concentration is 0.17M (Figure 3) higher quantities of peroxidase are needed than when the carbamide peroxide concentration is 0.09M (Figure 4). Adding peroxidase until a final activity of 4 units pyrogallol/ml is obtained hardly modifies the speed with which a 0.015M tetracycline solution is rendered colourless by the mouth wash. However, with a perioxidase activity of 20 units pyrogallol/ml the speed with which a tetracycline solution (0,015M) is rendered colourless by the mouth wash (0,17M, final concentration of carbamide peroxide) increases from approximately 70nKat to 95nKat owing to the presence of the peroxidase. Owing to the peroxidase this activation is even greater when the mouth wash concentration is less. Therefore, adding mouth wash (0.09M carbamide peroxide, final concentration) to the tetracycline solution (0.015M) gives rise to a de-staining speed of approximately 40nkat; adding 20 units of pyrogallol/ml of peroxidase increases this de-staining speed to approximately 70 nkat, and if the peroxidase concentration is 40 units pyrogallol/ml the de-staining speed increases to approximately 110nkat, much greater than when using only the mouth wash, even with concentrations double the strength of that used in this experiment.

### EXAMPLE 2

This example illustrates the bleaching action of a mouth wash designed on the basis of using peroxidase to de-stain teeth. The treatment time is long, equivalent to exposure periods of one night or a whole day.

The composition of the mouth wash and the peroxidase solution are as in example 1, in this case volumes of each solution came into contact with one another so that the final concentration in contact with the tooth was 1.5% carbamide peroxide and 120 units pyrogallol/ml of peroxidase. The mouth wash and the enzyme can be applied directly to the tooth outside the mouth. This was the case used in this example, although it can also be carried out by directly applying the mouth wash in the mouth, using preformed trays which only come into contact with the tooth crown thereby guaranteeing that the bleaching action occurs on the tooth.

Teeth de-staining was continued using a colorimeter for solids (Minolta trademark) with which the percentage brightness of the tooth was measured, on a scale of 0-100% from dark to light.

The teeth were in contact with the bleaching solutions for a long time so that each time the tooth brightness was measured, the bleaching solutions were renewed. The brightness measurement times, apart from the initial time with untreated teeth, were 8, 24, 48 and 72 hours after the start of the experiment.

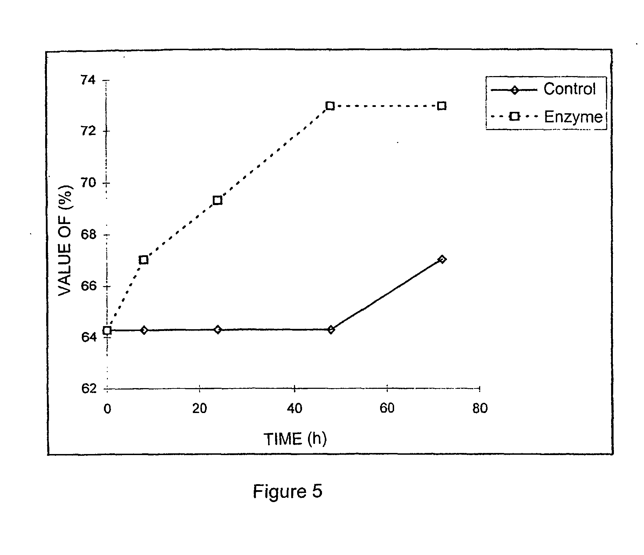

Tooth de-staining occurs first when the enzyme is present as opposed to when it is absent (Figure 5). Using as models two teeth that had initially the same brightness value (64%), one tooth was treated only with the mouth wash, whereas the enzyme solution was added to the other tooth. The tooth only treated with the mouth wash maintained its 64% brightness value after 8, 24 and 48 hours, being rendered slightly colourless after 72 hours when it presented a 66% brightness value. However, the tooth treated with mouth wash and peroxidase was already colourless after 8 hours and presented a 66% brightness value. The de-staining continued after 24 and 48 hours giving rise to brightness values of approximately 68% and 72%, respectively, the maximum bleaching effect obtained. No variation in the brightness value occurred between 48 and 72 hours.

### EXAMPLE 3

This example illustrates the action of a mouth wash designed on the basis of using peroxidase to de-stain teeth. The treatment time is short, only a few minutes and therefore it can be applied in the dentist surgery or as part of a daily dental hygiene routine.

The composition of the mouth wash and the peroxidase solution is the same as that in Example 1. The final concentrations of peroxidase and carbamide peroxide in contact with the tooth are the same as those in Example 1 and Example 2. These final concentrations can also be obtained from other initial concentrations of mouth wash and enzyme solution or, even from a dentifrice with a composition suitable for keeping the enzyme active, and from a mouth wash containing carbamide peroxide that can be used to rinse the teeth after cleaning them with the enzyme containing dentifrice. Also, the final peroxidase and carbamide peroxide concentrations can be obtained by preparing a gel that contains the active enzyme and another gel that contains the carbamide peroxide. In order to obtain the concentrations assayed in this example, before being applied to the tooth, the two gels shall be mixed in quantities appropriate for the concentration of the active ingredients thereof.

The peroxidase and the carbamide peroxide are in contact with the tooth for 5 minutes.

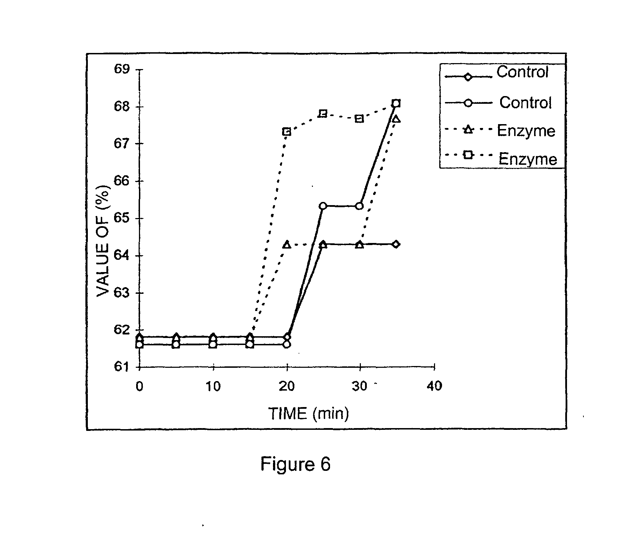

Continually treating teeth during 5-minute periods with carbamide peroxide and peroxidase will bleach them, whereas if only carbamide peroxide is used the bleaching effect is delayed and does not reach such high values (Figure 6). Starting with five teeth having an initial 62% brightness value, treating 3 teeth individually with the mouth wash and the enzyme solution and another two only with the mouth wash, the bleaching effect is noticeable in the teeth treated with peroxidase after 4 five-minute periods of treatment (teeth brightness values between 64% and 67%), whereas the teeth not treated with peroxidase show evident signs of the bleaching effect after 5 treatment periods (64% and 65% teeth brightness values). In successive periods of treatment with peroxidase the brightness value of the teeth continues to increase up to 68%-69%, whereas in the teeth not treated with enzyme, the bleaching effect stabilises at 64% or increases slightly to 68%. The tooth's varied response to the enzyme's bleaching action may be due to the fact that different types of stains are present on the teeth, although in each case, a more efficient bleaching effect was obtained when peroxidase was present.

## Claims

1. Dental bleaching product comprising carbamide peroxide and peroxidase.

2. Dental bleaching product according to claim 1, further comprising a buffering substance, preferably phosphate buffer.

3. Dental bleaching product according to claim 2, wherein the pH value of the reaction is buffered to a range between 4 and 9, preferably 6.5.

4. Dental bleaching product according to any of the claims 1 to 3, further comprising substances that activate said peroxidase.

5. Dental bleaching product according to any of the claims 1 to 4, wherein the peroxidase activity is a minimum of 10 units of pyrogallol, preferably a minimum of 20 units of pyrogallol.

6. Dental bleaching product according to claim 1, wherein said peroxidase is any enzymatic isoform of peroxidase, said peroxidase originating from a vegetable source, an animal source or a micro-organism, preferably radish.

7. Method for applying the dental bleaching product according to claim 1, comprising the steps of:
(d) keeping said bleaching compound and said enzyme separate until the moment the product is applied;
(e) mixing said bleaching compound and said peroxidase immediately before applying the product; and
(f) applying the mixture of said bleaching compound and said peroxidase to the teeth.

8. Method for applying the dental bleaching product according to claim 1, comprising the steps of:
(d) keeping said bleaching compound and said enzyme separate until the moment the product is applied;
(e) first applying said bleaching compound to the teeth; and
(f) immediately applying said peroxidase.

9. Method for applying the dental bleaching product according to claim 1, comprising the steps of:
(d) keeping said bleaching compound and said enzyme separate until the moment the product is applied;
(e) first applying said peroxidase to the teeth; and
(f) immediately applying said bleaching compound.

10. Formulation comprising a dental bleaching product according to claim 1, wherein the peroxidase is dissolved or emulsified in a carrier.

11. Formulation comprising a dental bleaching product according to claim 1, wherein peroxidase is immobilised or microencapsulated in a carrier.

12. Formulation according to claim 10 or 11, wherein said carrier is a mouth wash, a dentifrice or a gel.

13. Use of a dental bleaching product according to claim 1 for dental bleaching.

14. Use of peroxidase as a catalyst in dental de-staining reactions, which use a carbamide peroxide bleaching agent.

## Patentansprüche

1. Dentalbleichprodukt, umfassend Carbamidperoxid und Peroxidase.

2. Dentalbleichprodukt nach Anspruch 1, ferner umfassend eine Puffersubstanz, vorzugsweise Phosphatpuffer.

3. Dentalbleichprodukt nach Anspruch 2, wobei der pH-Wert der Reaktion auf einen Bereich zwischen 4 und 9, vorzugsweise 6,5, gepuffert ist.

4. Dentalbleichprodukt nach einem der Ansprüche 1 bis 3, ferner umfassend Substanzen, die die Peroxidase aktivieren.

5. Dentalbleichprodukt nach einem der Ansprüche 1 bis 4, wobei die Peroxidaseaktivität ein Minimum von 10 Einheiten Pyrogallol, vorzugsweise ein Minimum von 20 Einheiten Pyrogallol aufweist.

6. Dentalbleichprodukt nach Anspruch 1, wobei die Peroxidase eine jegliche enzymatische Isoform von Peroxidase ist, wobei die Peroxidase von einer pflanzlichen Quelle, einer tierischen Quelle oder einem Mikroorganismus, vorzugsweise Rettich, abstammt.

7. Verfahren zum Auftragen des Dentalbleichprodukts nach Anspruch 1, umfassend die Schritte:
(d) Getrennthalten der Bleichverbindung und des Enzyms bis zu dem Zeitpunkt, an dem das Produkt aufgetragen wird,
(e) Mischen der Bleichverbindung und der Peroxidase direkt vor einem Auftragen des Produkts und
(f) Auftragen des Gemisches der Bleichverbindung und der Peroxidase auf die Zähne.

8. Verfahren zum Auftragen des Dentalbleichprodukts nach Anspruch 1, umfassend die Schritte:
(d) Getrennthalten der Bleichverbindung und des Enzyms bis zu dem Zeitpunkt, an dem das Produkt aufgetragen wird,
(e) zuerst Auftragen der Bleichverbindung auf die Zähne und
(f) direktes Auftragen der Peroxidase.

9. Verfahren zum Auftragen des Dentalbleichprodukts nach Anspruch 1, umfassend die Schritte:
(d) Getrennthalten der Bleichverbindung und des Enzyms bis zu dem Zeitpunkt, an dem das Produkt aufgetragen wird,
(e) zuerst Auftragen der Peroxidase auf die Zähne und
(f) direktes Auftragen der Bleichverbindung.

10. Formulierung, umfassend ein Dentalbleichprodukt nach Anspruch 1, wobei die Peroxidase in einem Träger gelöst oder emulgiert ist.

11. Formulierung, umfassend ein Dentalbleichprodukt nach Anspruch 1, wobei die Peroxidase in einem Träger immobilisiert oder mikroeingekapselt ist.

12. Formulierung nach Anspruch 10 oder 11, wobei der Träger ein Mundwasser, eine Zahnpaste oder ein Gel ist.

13. Verwendung eines Dentalbleichprodukts nach Anspruch 1 zum Zahnbleichen.

14. Verwendung von Peroxidase als einem Katalysator in Dentalentfärbungsreaktionen, die ein Carbamidperoxidbleichmittel verwenden.

## Revendications

1. Produit pour le blanchiment dentaire comprenant du peroxyde d'urée et de la peroxydase.

2. Produit pour le blanchiment dentaire selon la revendication 1, comprenant en outre une substance de tamponnage, de préférence un tampon à phosphates.

3. Produit pour le blanchiment dentaire selon la revendication 2, dans lequel la valeur de pH de la réaction est tamponnée à une fourchette allant de 4 à 9, de préférence à 6,5.

4. Produit pour le blanchiment dentaire selon l'une quelconque des revendications 1 à 3, comprenant en outre des substances qui activent ladite peroxydase.

5. Produit pour le blanchiment dentaire selon l'une quelconque des revendications 1 à 4, dans lequel l'activité de la peroxydase est un minimum de 10 unités de pyrogallol, de préférence un minimum de 20 unités de pyrogallol.

6. Produit pour le blanchiment dentaire selon la revendication 1, dans lequel ladite peroxydase est une isoforme enzymatique de peroxydase, ladite peroxydase provenant d'une source végétale, d'une source animale, ou d'un microorganisme, de préférence de radis.

7. Méthode d'application du produit pour le blanchiment dentaire selon la revendication 1, comprenant les étapes consistant à :
(d) maintenir ledit composé blanchissant et ledit enzyme séparés jusqu'au moment où le produit est appliqué ;
(e) mélanger ledit composé blanchissant et ladite peroxydase juste avant d'appliquer le produit ; et
(f) appliquer le mélange dudit composé blanchissant et de ladite peroxydase sur les dents.

8. Méthode d'application du produit pour le blanchiment dentaire selon la revendication 1, comprenant les étapes consistant à :
(d) maintenir ledit composé blanchissant et ledit enzyme séparés jusqu'au moment où le produit est appliqué ;
(e) appliquer d'abord ledit composé blanchissant sur les dents ; et
(f) appliquer immédiatement ladite peroxydase.

9. Méthode d'application du produit pour le blanchiment dentaire selon la revendication 1, comprenant les étapes consistant à :
(d) maintenir ledit composé blanchissant et ledit enzyme séparés jusqu'au moment où le produit est appliqué ;
(e) appliquer d'abord ladite peroxydase aux dents ; et
(f) appliquer immédiatement ledit composé blanchissant.

10. Formulation comprenant un produit pour le blanchiment dentaire selon la revendication 1, dans laquelle la peroxydase est dissoute ou émulsifiée dans un support.

11. Formulation comprenant un produit pour le blanchiment dentaire selon la revendication 1, dans laquelle la peroxydase est immobilisée ou microencapsulée dans un support.

12. Formulation selon la revendication 10 ou 11, dans laquelle ledit support est un bain de bouche, un dentifrice ou un gel.

13. Utilisation d'un produit pour le blanchiment dentaire selon la revendication 1, pour le blanchiment dentaire.

14. Utilisation de peroxydase en tant que catalyseur dans des réactions de détachage dentaire qui utilisent un agent blanchissant au peroxyde d'urée.
